Europäisches Patentamt

European Patent Office

Office européen des brevets

(11) Publication number: **0 479 280 A1**

## EUROPEAN PATENT APPLICATION

(21) Application number: **91116849.0**

(22) Date of filing: **02.10.91**

(51) Int. Cl.⁵: **A61K 35/12**, A61K 39/00, A61K 37/02

(30) Priority: **03.10.90 US 592470**
**03.10.90 US 592471**

(43) Date of publication of application:
**08.04.92 Bulletin 92/15**

(84) Designated Contracting States:
**AT BE CH DE DK ES FR GB GR IT LI LU NL SE**

(71) Applicant: **YEDA RESEARCH AND DEVELOPMENT LTD.**
**P.O. Box 95**
**Rehovot(IL)**

(72) Inventor: **Cohen, Irun R.**
**11, Hankin Street**
**Rehovot(IL)**
Inventor: **Lohse, Ansgar W.**
**Apelstrasse 6**
**W-6500 Mainz(DE)**
Inventor: **Mekori, Yoseph A.**
**1, Hakeshet Street, Kfar Saba**
**IL-44414(IL)**

(74) Representative: **Vossius & Partner**
**Siebertstrasse 4 P.O. Box 86 07 67**
**W-8000 München 86(DE)**

(54) **T cell vaccination for prevention and treatment of allergy or graft rejection.**

(57) Compositions for prevention or treatment of allergy or graft rejection comprising activated T lymphocytes specific either for the antigen provoking said allergy or for any antigen provoking graft rejection, membrane preparations of such T lymphocytes, T cell receptor proteins of such T lymphocytes, or peptides of about 5-30 amino acids corresponding to a part of these T cell receptor proteins, are disclosed. The compositions comprise said T lymphocytes which are activated **in vitro** by a specific antigen or by a T cell mitogen and may optionally be further treated to produce membrane aggregation prior to their use.

EP 0 479 280 A1

The present invention relates to compositions comprising activated T lymphocytes, membrane preparations thereof, T cell receptor proteins of said T lymphocytes, or peptides derived from such T cell receptor proteins, useful for preventing or treating graft rejection or allergy.

Three areas in which the immune system acts detrimentally to the host are hypersensitivity, autoimmunity and tissue transplantation. Graft rejection between unrelated individuals has long been known to be due to the action of the adaptive immune system, and manifests its two key features, specificity and memory. It is believed that the capacity to reject foreign grafts, which stands as a major barrier to successful clinical transplantation, is a side effect of the immune system's evolution to recognize and destroy virally-altered cells.

Allograft reactions are mediated by clones of effector T lymphocytes that recognize cell surface histocompatibility antigens expressed on cells in the graft. Such histocompatibility antigens may be "major", that is, encoded by class I or class II genes of the major histocompatibility omplex (MHC), such as HLA in man or H-2 in mouse. Alternatively, minor histocompatibility antigens are antigens presented to the recipient's T lymphocytes in the context of MHC gene products expressed on the recipient's antigen-presenting cells.

Transplantation of kidneys, bone marrow, skin, liver, lung, heart, and endocrine tissues has become increasingly important in medicine. Acceptance of a graft depends on a variety of donor and host factors, with histocompatibility between the donor and the host being among the most important. In the absence of compatibility, it is generally necessary to suppress the immune system of the host in order to overcome the potent immune response that leads to graft rejection.

Most commonly, antigen non-specific approaches to immunosuppression have been utilized. This includes the use of immunosuppressive drugs and other ways to reduce T cell function. Classic drugs used for this purpose are corticosteroids and azathioprine. These drugs produce a number of unwanted side effects, such as increased susceptibility to infections by certain bacteria, viruses, and protozoans, due to generalized suppression of the immune system. Other therapies directed at T cells have included anti-lymphocyte sera or globulins given at the time of transplantation, and lymphoid irradiation prior to transplantation. However, these approaches also leave the patient susceptible to infections. When infection occurs, treatment must be halted, often leading to development of allogeneic reactions by the immune system and ultimate rejection.

Antigen-specific forms of immunosuppression have been the ideal toward which much research effort has been expended. One such approach is immunological enhancement, wherein non-cytotoxic antibodies to the graft alloantigens are provided and thereby prevent either stimulation of or cytotoxic action of antigen-specific T lymphocytes. Another approach is directed to immunological tolerance and involves the induction of suitable anti-idiotypic antibodies prior to grafting, which would specifically inhibit limited clones of T cells from acting.

More recent approaches have involved the use of monoclonal antibodies directed against structures on the surface of T cells including T cell receptors or their associated structures (Hirsch, R. et al., J. Immunol. **140**:3766 (1988)), or receptors for interleukins (Granstein, R.D. et al., J. Immunol. **136**:898 (1986)) which must be triggered during the course of a T lymphocyte response. These approaches have been shown to have potential utility. Additionally, antibodies to dendritic cells, which are potent stimulators of immunity and are passenger cells in most tissue and organ grafts, may inhibit rejection (Faustman, D.L. et al., Proc. Natl. Acad. Sci. USA **81**:3864-68 (1984)).

However, most of the newer approaches have not yet proved to be the hoped-for panacea, many remain experimental, and the art remains desperately in need of new aggressive and comprehensive therapeutic approaches for improved success in a variety of clinical transplantation settings.

Immediate hypersensitivity is a form of immunity, or allergy, which belongs to the family of antibody-mediated effector responses. IgE antibodies play a central role in the pathogenesis of immediate hypersensitivity reactions. This class of antibodies binds to specialized high affinity Fc receptors on mast cell and basophils. When the bound antibodies are cross-linked by the antigen (or allergen), cellular degranulation of the mast cells and basophils occurs, with the release of vasoactive amines, proteolytic enzymes, proteoglycans, and products of arachidonic acid metabolism. These powerful chemical mediators trigger a sequence of physiologic events in various organs, resulting in the well-known set of allergic symptoms such as anaphylaxis, allergic rhinoconjunctivitis, asthma, urticaria-angioedema, atopic dermatitis, and the like.

Contact sensitivity is a form of antigen-specific delayed-type hypersensitivity (DTH) to substances such as chemicals which bind to and chemically react with self constituents. Contact sensitivity reactions are mediated by clones of effector T lymphocytes that recognize chemical antigens presented in the context of class II major histocompatibility (MHC) gene products expressed on cells in the skin. In its clinical manifestation, this class of response is known as contact dermatitis. Although often treatable with corticosteroids, it causes great discomfort and is frequently disabling.

Therapy of immediate hypersensitivity disorders, such as allergic rhinitis, has included the use of various pharmacologic agents, such as disodium chromoglycate, steroids, theophylline, and others. However, since the allergic process persists, none of the agents described cure the allergic state, and must therefore be taken chronically, often producing side effects or dependence.

Immunotherapy involving injection of increasing amounts of antigen (allergen) for allergic disorders has been practised since the beginning of the century. Since aqueous extracts of allergens currently in use are immunogenic, they can cause serious side effects, and the amount of antigen that can be delivered to the patients is limited. Therefore, attempts have been made to modify allergens to decrease their allergenicity without affecting their ability to produce the desired reduction in allergen-specific IgE. Trials of such modified allergens in allergic patients have produced disappointing results. In animal models, relatively large doses of modified allergens had to be given repeatedly, or hypersensitivity would return.

None of the clinical approaches to allergy, of either the IgE-mediated immediate hypersensitivity type or the contact sensitivity type, have been targeted to antigen-specific T lymphocytes.

The role of antigen-specific T cells in contact sensitivity, mentioned above, is central to the induction process (see, for example, Claman, H.N. et al., Adv.Immunol. 30:121 (1980)). In IgE-mediated allergy, T cells may be involved at several different levels. First, IgE synthesis is influenced predominantly by T suppressor cells, that limit IgE production, and involves a balance between products of T helper cells, including glycoproteins such as interleukin 4 (IL-4), and suppressive glycopeptides (Ishizaka, K., J.Immunol. **135**:1 (1985); Finkelman, F.D. et al., **141**:2335 (1988)). Secondly, T cell-derived lymphokines, such as IL-3 and IL-4, influence the growth and differentiation of mast cells, the effector cells of allergic (and many inflammatory) reactions (Nabel et al., Nature **291**:332 (1981)). Thirdly, T cells can modulate various mast cell reactions, including the release of mediators (Dy, M. et al., J.Immunol. **136**:208 (1986); Thuesson et al., J.Immunol. **123**:626 (1979)). Indeed, T cells from allergic patients show a significant proliferative response upon stimulation with the specific allergen (Ledru et al., Clin.Exp.Immunol. **73**:198 (1988)).

T cell vaccination is a form of immunotherapy which was originally demonstrated to be effective against a variety of experimental autoimmune diseases. T cell vaccines comprised antigen-specific lines or clones of T lymphocytes which were attenuated by gamma or X-irradiation (Maron, R. et al., J. Immunol. **131**:2316 (1983); Ben-Nun, A. et al., Nature **292**:60 (1987); Cohen, I.R., Immunol. Rev. **94**:5 (1986)). Rats or mice receiving irradiated T lymphocytes reactive to myelin basic protein (MBP) or to thyroglobulin acquired resistance to subsequent attempts to induce experimental autoimmune encephalomyelitis (EAE) (Ben-Nun et al., supra), or thyroiditis (Maron et al., supra), respectively. Vaccination against adjuvant arthritis in rats was obtained using an antigen-specific T cell line which did not have to be attenuated by irradiation (Holoshitz, J. et. al., Science **219**:56 (1983)). Some virulent (disease-inducing) clones of T cells could induce resistance rather than disease when administered at cell doses below the threshold needed for disease induction (such as $10^4$ or fewer cells) (Lider, 0. et al., Science **239**:181 (1988)).

For effective vaccination by T cells (as was the case for induction of disease by T cells) they must be activated before administration (Naparstek, Y. et al., Eur. J. Immunol. **13**:41 (1983)). T cells lines or clones neither caused disease nor induced resistance if they had not been cultured with their specific antigen or with a polyclonal activator of T cells, such as concanavalin A, before administration to recipient animals. Among other effects, this **in vitro** activation produced changes in T cell membrane components, modified T cell traffic in the body, and induced the expression of enzymes. However, the elements of activation critical for the capability for T cell vaccination are not yet well-defined.

The capacity of activated T cells to vaccinate has been enhanced by treating them in ways that caused aggregation of membrane components (Cohen, 1986, supra; Lider, 0. et al., Proc. Nat. Acad. Sci. USA **84**:4577 (1987)). A rat T cell clone that otherwise could not vaccinate, upon treatment with hydrostatic pressure, with a chemical cross-linking agent such as glutaraldehyde, or with a cytoskeletal disrupting agent such as cytochalasin B, acquired the capacity to prevent adjuvant arthritis and to induce rapid remission of already established disease. Similarly, fragments of T cells so treated were also active. Treatment with glutaraldehyde could also augment the ability of uncloned populations of T cells taken from lymph nodes of antigen-primed animals to vaccinate recipients. (See: Cohen, 1986, supra; Cohen, I.R. et al., U.S. Patent No. 4,634,590 (January 6, 1987); Stanford et al., U.S. Patent No. 4,716,038 (December 29, 1987); Cohen, I.R. et al., European Patent Publication EP 261648 ((3/30/80); Cohen, I.R., European Patent Publication EP 291046 (November 17, 1988)).

More recently, therapeutic approaches to autoimmune diseases, utilizing the EAE model, have been expanded to include the use of peptide vaccines derived from the T cell receptor (TCR) of MBP-specific T cells, in place of the T cells themselves. Vandenbark, A.A., et al. (Nature **341**: 541-544 (1989) showed that immunization with a TCR-like peptide of 20 amino acids corresponding to the CDR2 region of the TCR $\beta$

chain induced a state of anti-idiotypic (or receptor-specific immunity) which was protective against EAE: furthermore, T cells from such TCR peptide-immunized animals passively transferred counter-autoimmune effects. Howell, M.D. et al. (Science **246**:668-671 (1989)) disclosed synthetic peptides corresponding to idiotypic determinants of the TCR common to a number of encephalitogenic T cell lines in mice and rats. Peptides of 8, 9, or 11 amino acids, corresponding to the $\beta$ chain VDJ region or the J$\alpha$ regions proved effective as vaccines for either preventing or reducing the severity of EAE in Lewis rats produced by guinea pig MBP in adjuvant. This approach was said by the authors to be preferable to the use of either mAbs to the TCR, attenuated encephalitogenic T cell lines as vaccines, or the transfer of idiotype-specific regulatory T cells. The authors also suggested the use of this approach in modulating T helper cells involved in the production of pathogenic autoantibodies, T cell lymphomas, and "other pathogenic conditions mediated by specific, oligoclonal T cells." (page 670 col. 1, para 2). Neither of these references, however, contemplates the use of TCR derived peptides as vaccines in the treatment of graft rejection responses.

The mechanism of resistance to the pathologic autoimmune response induced by whole T cell or TCR peptide vaccination seems to involve generation of anti-idiotypic T cells in the vaccinated animal. T cells of both CD4 and CD8 classes, obtained from vaccinated animals which had become disease-resistant, showed specific responsiveness to the T cell clone (Lider et al., Science, **244**:820 (1989)), or its TCR peptide, used for vaccination. Indeed, a CD8[+] T cell clone which was responsive to an anti-MBP T cell was able to mediate resistance to adoptive transfer of encephalomyelitis (Sun, D. et al., Nature **332**:843 (1988)). However, additional protective mechanisms are also thought to be set into motion by T cell vaccination (Lohse et al., Science **244**:820 (1989)). It is thought that aggregating the antigen-specific T cell receptors (TCR) in the membrane by hydrostatic pressure, cross-linking agents, or cytoskeletal disrupting agents, as discussed above, or by gamma- or X-irradiation or treatment with mitomycin C, potentiates their ability to induce anti-idiotypic responses.

In addition to the anti-idiotypic T cells, T cell vaccination induces other regulatory T cells that recognize activation markers on T cells, irrespective of their antigen receptors. This type of regulatory T cell response has been termed anti-ergotypic (Lohse et al., Science, **244**:820 (1989)). Thus, anti-idiotypic and anti-ergotypic T cells combine to control the activities of the autoimmune T cells responsible for the particular disease.

Figure 1 is a histogram showing the effects of vaccinating mice with glutaraldehyde-treated immune lymph node cells (I-LNC) on the development of contact sensitivity to dinitrofluorobenzene (DNFB) induced by skin painting.

Figure 2 is a histogram showing the effects of vaccinating mice with glutaraldehyde treated immune lymph node cells (I-LNC) on the development of contact sensitivity to oxazolone (Ox) induced by skin painting.

Figure 3 is a histogram showing the antigen specificity of vaccination with immune lymph node cells specific for Ox (I-LNC(Ox)).

Figure 4 is a histogram showing the specificity of vaccination in an adoptive transfer protocol, wherein the non-immune recipient mice were vaccinated with I-LNC prior to transfer of immune lymphocytes.

Figure 5 is a histogram showing an anti-idiotypic delayed type hypersensitivity (DTH) response to vaccination with I-LNC.

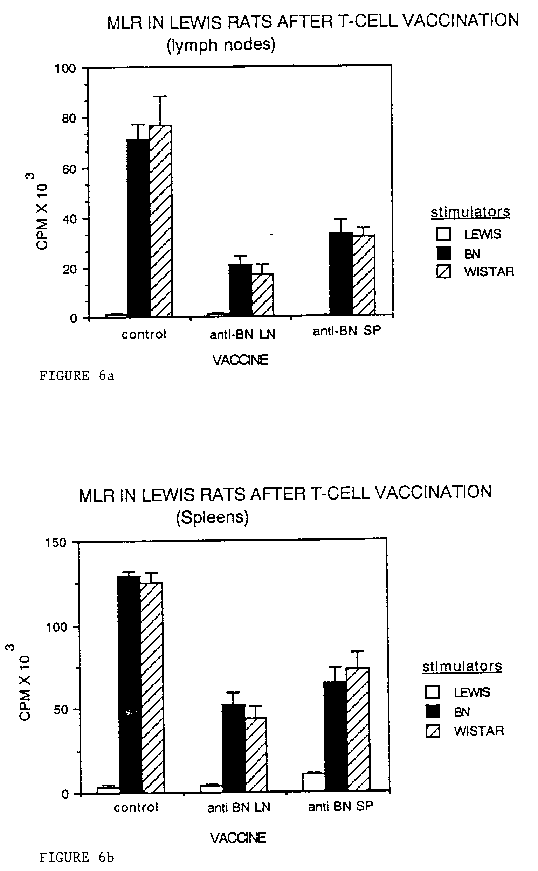

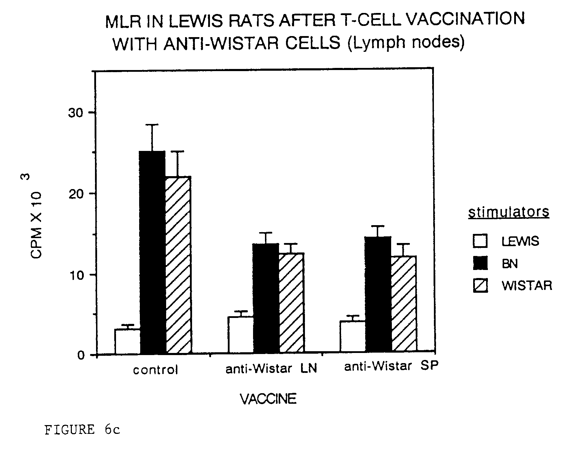

Figure 6a, 6b and 6c are graphs showing the mixed lymphocyte reaction (MLR) of lymphocytes from rats treated with T cell vaccines. Lewis rats were vaccinated with Lewis spleen or lymph node (LN) cells taken from rats primed with histocompatibility antigens in the form of allogeneic cells (either allogeneic spleen or LN). Figure 6a: MLR of LN cells from recipients of Lewis anti-BN LN or Lewis anti-BN spleen cell vaccines. Figure 6b: MLR of spleen cells from recipients of Lewis anti-BN LN or Lewis anti-BN spleen cell vaccines. Figure 6c: MLR of LN cells from recipients of Lewis anti-Wistar LN or Lewis anti-Wistar spleen cell vaccines.

Figure 7 is a graph showing the MLR of cells from BALB/c mice vaccinated with BALB/c LN or spleen cells from mice immunized with allogeneic C57BL/6 cells.

Figure 8 is a graph showing the MLR of cells from BALB/c mice primed with C57BL/6 spleen cells which were either untreated or activated **in vitro** with the mitogen, Con A.

Figure 9 is a graph showing an anti-ergotypic response wherein Lewis rats were primed with Con A-activated Lewis spleen cells and their spleen cells were tested for MLR with control or Con A activated stimulator cells.

Figure 10 is a graph showing suppression of MLR of naive cells by spleen cells from vaccinated rats.

Figure 11 is a graph showing survival of heart allografts in rats vaccinated with T cells activated **in vitro** against donor histocompatibility antigens.

Figure 12 is a graph showing survival of heart allografts in rats vaccinated with T cells which had been primed **in vivo** by skin grafting donor-type skin, followed by activation **in vitro**.

Figure 13 is a graph showing that T cell vaccination prolongs survival of BN cardiac allografts in Wistar rats.

It has now been found according to the present invention that antigen-specific T cells, whether a cell line, or clone, or even unfractionated populations of lymphocytes taken from an allergic subject, upon activation **in vitro**, are capable of serving as antigen-specific vaccines which lead to reduction of allergic responses in that subject.

It has further been found according to the present invention that T cells responsive to foreign tissue antigens (alloantigens or allogeneic MHC antigens), whether a cell line, or clone, or even unfractionated populations of lymphocytes taken from a subject, upon activation **in vitro** to respond to foreign cells, are capable of serving as vaccines which lead to reduction of immune reponses to foreign MHC antigens and suppression of graft rejection in that subject. These T cell vaccines are effective when the T cells are activated with any alloantigen, and need not be specific for the MHC alleles of the graft tissue donor.

The present invention is thus directed to compositions useful for preventing or treating allergy or graft rejection in a subject comprising T lymphocytes specific for the antigen provoking said allergy or responsive to any antigen provoking graft rejection activated **in vitro** with the antigen or with a mitogen, and optionally subjected to treatment which produces membrane aggregation. Such treatment of the cell includes exposure to a chemical cross-linking agent, hydrostatic pressure, a cytoskeletal disrupting agent, attenuation with X- or gamma irradiation or mitomycin C treatment, or a combination of the above.

In one embodiment, the composition used to induce immunological tolerance to the antigen comprises glutaraldehyde-treated immune lymph node cells (I-LNC), which can prevent or treat graft rejection or allergic reactions.

The invention further includes compositions comprising subcellular membrane preparations of the activated, optionally treated T cells described above, or T cell receptor (TCR) proteins of such cells, or peptides of about 5-30 amino acids corresponding to the sequence of a portion of the above TCR proteins.

The invention is also directed to a method for producing a composition useful for the prevention or treatment of graft rejection or of allergy in a subject, which composition comprises activated T lymphocytes optionally subjected to treatment which produces membrane aggregation, the T lymphocytes being either specific to the antigen provoking the allergy or responsive to alloantigens such as an alloantigen which provokes graft rejection. The method comprises: (a) obtaining the alloantigen-responsive or the antigen-specific T lymphocytes from an animal or from a cell culture; (b) treating the T lymphocytes **in vitro** with an activating agent, thereby activating the T lymphocytes; and (c) optionally treating the activated T lymphocytes to cause membrane aggregation, e.g., with a chemical cross-linking agent, hydrostatic pressure or a cytoskeletal disrupting agent and/or to cause attenuation with gamma- or X-irradiation or mitomycin C, thereby producing the composition. Also included is a method which further involves preparation of subcellular membrane preparations or TCR proteins or peptides from the above TCR proteins.

In one aspect, the compositions of the invention are T cell vaccines useful for the treatment of graft rejection, comprising T cells responsive to alloantigens which provoke graft rejection. The alloantigens may be derived from any foreign tissue. In a preferred embodiment, the alloantigens are derived from the graft donor, but also alloantigens derived from non-graft donors are encompassed by the invention. Vaccination with such T cells causes a significant alloantigen specific inhibition of mixed lymphocyte culture (MLC) reactivity **in vitro** and significant inhibition of heart allograft rejection **in vivo**. This induction of antigen-specific immunological tolerance is associated with an anti-idiotypic response to the T cell vaccine.

In another aspect, the compositions of the invention are T cell vaccines useful for the treatment of allergic reactions comprising T cells specific to the antigen provoking said allergic reations. Vaccination with such T cells causes a significant antigen-specific inhibition of contact sensitivity, and significant inhibition of an antigen specific IgE antibody response. This induction of antigen-specific immunological tolerance is associated with an anti-idiotypic response to the T cell vaccine.

The results reported herein indicate that T cell vaccination leads to immunological tolerance, indicated by the induced lack of immunologically specific reactivity to the antigen in question or to simple chemical structures, such as haptens. Even though their chemical structure is simple, reactive haptens in contact sensitivity reactions are rendered immunogenic through chemical combination with self "carrier molecules"- (such as MHC class II molecules on various types of antigen presenting cells, including Langerhans cells in the skin). Also shown is anti-ergotypic suppression. By the term "ergotypic" is intended a response directed against activation markers on T cells (Lohse et al., supra), as opposed to TCR or MHC molecules.

It would not be expected that neither such alloantigen-responsive tolerance for structures which are not "self" nor that such antigen-specific tolerance for simple chemical structures which are not "self", would occur merely from knowledge of the effectiveness of T cell vaccination against autoantigens in autoimmune diseases. First, there is a major difference in the nature of these histocompatibility antigens, in case of graft rejection, or of these foreign chemical antigenic moieties, in the case of allergic reactions, and the autoantigens which have been analyzed.

Second, there is a major difference in how the immune system is "arrayed" with respect to its idiotypic-anti-idiotypic network. With self antigens of the type to which autoimmune diseases are directed, an idiotypic network has had time to develop, and is presumably in place when the dysregulation leading to the disease occurs. Thus, perturbation of such a well-developed network of interacting cells and self structures by vaccinating with a population of specific T cells bearing a particular T cell receptor is quite different from perturbing the immune system and affecting the response to a "new" antigen (such as a foreign histocompatibility antigen or a chemical hapten which causes contact sensitivity). In the former case, anti-idiotypic cells are present when the T cell vaccine is administered, and presumably these cells respond to the T cell receptors of the vaccinating cells, thereby modifying the network and resulting in a new state in which the autoimmune response is inhibited (Cohen, I.R., In: Cold Spring Harbor Symp. Quant. Biol. Vol.LIV, Cold Spring Harbor Laboratory Press, Cold Spring Harbor, New York, 1989, pp. 879-887).

In the case of a new antigen, such as a foreign histocompatibility antigen or an allergen capable of inducing IgE-mediating allergy or T-cell mediated contact sensitivity, no analogous idiotypic network is in place. The vaccinating T cells, specific for that antigen presumably encounter a totally different milieu. There should be few or no pre-existing anti-idiotypic cells specific for the T cell receptors of the vaccinating preparation. Therefore, it was highly unexpected to find that a similar strategy of T cell vaccination using T cells responsive to any antigen which provokes graft rejection proved effective in preventing or reversing allograft rejection, and that using T cells specific for the antigen provoking an allergic reaction proved effective in preventing or reversing contact sensitivity and IgE-mediated allergic responses.

By the term "T lymphocyte" or "T cell" is intended a cell of the lymphocyte lineage which is thymus-derived in origin, as is well known in the art. See, for example, Roitt, I., Essential Immunology, 6th Ed., Blackwell Scientific Publications, Oxford (1988): Roitt, I. et al., Immunology, C.V. Mosby Co., St.Louis, MO (1985): Klein, J., Immunology: The Science of Self-Nonself Discrimination, John Wiley & Sons, New York, NY (1982)). As is known in the art, a variety of defined subsets of T cells exist in the body, such as CD4 + T helper cells, CD8 + cytotoxic T cells or suppressor cells, and the like. For the purposes of the present invention, T lymphocytes spanning the subset categories are useful, and there is no established preference for T cells of any given subset.

The term "immunological tolerance" as used herein refers to a state in which an immune response to a specific antigen is depressed due to a manipulation of the responding subject in an antigen-specific manner. Tolerance may be induced and maintained by any of a number of mechanisms, including blocking antibodies, suppressor cells, clonal deletion or clonal anergy, etc., which are well-known in the art (see: Roitt, supra; Klein, supra). The invention is not to be limited by any particular mechanistic explanation, and the term "tolerance" is used herein in its broadest generic sense, as defined above.

The T lymphocytes of the present invention may be derived from an established T cell line or clone derived from the subject to be treated and maintained in cell culture, or may be taken from the blood, lymph or organized lymphatic tissue of the subject. By the term "organized lymphatic tissue" is intended any tissue or organ which contains large collections of lymphocytes, including, but not limited to, thymus, bone marrow, spleen, lymph node, and gut-associated lymphatic tissue (Roitt, supra: Roitt et al., supra; Klein, supra). The T cells are taken directly from a subject who is to be treated for graft rejection, or for a specific allergic reaction or disease.

A preferred source of T cells according to the present invention for prevention or treatment of graft rejection are peripheral blood lymphocytes of the recipient. Alternatively, T cells may be obtained from the circulation of a subject who was engrafted and is rejecting the graft.

A preferred source of T cells for the present invention for the prevention or treatment of allergic reactions is the site of an ongoing antigen-specific response, such as a draining lymph node in a subject immunized subcutaneously, intradermally or intracutaneously, or from the circulation of such a subject.

The T cells must be activated prior to administration. T cells typically do not induce resistance if they have not been exposed to an antigen to which they are specific or to a polyclonal T cell activator, such as the mitogen concanavalin A, before being administered to recipient subject.

By the term "activated T lymphocyte or T cell," as used herein, is intended a T lymphocyte which has been exposed to an activating agent, preferably **in vitro**. Preferred activating agents for the present invention include the specific antigen which provokes the allergic reaction, or an alloantigen, in the form of allogeneic cells such as blood leucocytes from the prospective organ or tissue donor. Alternatively, a polyclonal activator such as a mitogen, capable of inducing a response by that T cell, may be used. Such responses include a large number of intermediary metabolic changes, induction of macromolecular synthesis, such as DNA, RNA or protein synthesis, cell division, and the like, as is well-known in the art.

Suitable antigen-nonspecific agents capable of activating T cells are known in the art and include, but are not limited to, mitogens such as concanavalin A, phytohemagglutinin, and pokeweed mitogen. Additional activating agents are antibodies to T cell-surface structures, including but not limited to, antibodies to the CD3 cell-surface molecule, antibodies to the CD2 cell-surface molecule, antibodies to the CD28 cell-surface molecule, and the natural ligands of CD2 or CD28. Other activating agents include phorbol esters, such as phorbol myristate acetate, or a combination of a phorbol ester and a calcium ionophore, such as ionomycin.

Also intended as T cell activating agents are antibodies to the T cell receptor chains, specific for either the constant or the variable portions of those chains.

The T lymphocyte activation step of the present invention may or may not include the addition of T cell growth factors or stimulatory factors, such as, for example, IL-1, IL-2 or I-L4, to the culture medium for part or all of the activation interval.

Activation, among other effects, produces changes in T cell membrane components, modifies T cell traffic in the body and induces expression of enzymes. Activation is preferably performed **in vitro**. In the case of graft rejection it is carried out by incubating the T cells taken from the graft recipient with cells bearing foreign histocompatibility antigens, or alternatively, T cells can be taken from an immunized recipient, such as an individual who is rejecting a graft from the same donor or even from an unrelated donor, at a time when the lymphocytes express the activated state **in vivo**. In the case of allergy, activation is preferably performed by incubating the T cells taken from the subject with the specific antigen as activating agent, or alternatively, T cells can be taken from the immunized subject at a time when he expresses the activated state **in vivo**, such as from an allergic individual subjected to exposure to the allergen. In these cases, sufficient activation may have occurred and further **in vitro** activation may not always be necessary.

The capacity of activated T cells to vaccinate may be enhanced by treating the T cells in ways that cause aggregation of membrane components (Cohen, 1986, supra: Cohen, I.R. et al., U.S. Patent No. 4,634,590 (January 6, 1987): Stanford et al., U.S. Patent No. 4,716,038 (December 29, 1987): Cohen, I.R. European Patent Publication EP 261648 ((3/30/80): Cohen, I.R., European Patent Publication EP 291046 (November 17, 1988).

A preferred way of achieving such membrane aggregating effects is by the use of a chemical cross-linking agent, which augments the ability of T cells to vaccinate. The cross-linking agents which cause chemical aggregation of the cell membrane components, and which are useful in the present invention, include, but are not limited to, formaldehyde, glutaraldehyde, and the furocoumarins, also known as psoralens. Examples wherein glutaraldehyde is employed as the cross-linking agent are provided below.

Another means to achieve the membrane aggregation effect is the use of hydrostatic pressure, described in the Cohen patent publications cited above. In one embodiment of this invention, a combination of hydrostatic pressure followed by treatment with a chemical cross-linking agent is used.

Moreover, the pressure-treated or cross-linked, activated T lymphocyte cells of the present invention may be treated with a disrupting agent capable of causing the cell's cytoskeleton to dissociate, e.g., cytochalasin and colchicine.

In addition to the membrane aggregating treatments described above, or as a separate step, treatment to attenuate the T lymphocytes, including gamma- or X-irradiation, or treatment with mitomycin C, by methods well-known in the art, may also be used according to the invention (Ben Nun, A. et al., supra: Holoshitz, Y. et al., supra).

The scope of the present invention is intended to encompass subcellular membrane material from the intact T cell compositions of the invention. The membrane preparation may be produced according to methods well known in the art. The methods described by Cohen, I.R. et al. (U.S. Patent 4,634,590 and European Patent Publication EP 261648) can be used. For example, subcellular membrane components may be obtained by applying hydrostatic pressure to untreated T cells or to cross-linked or cytoskeletal disrupted T cells, for a time and at a pressure sufficient to cause shedding of membrane components. Both the soluble and insoluble components obtained after centrifugation may be used.

The peptides of the present invention comprise sequences of about 5-30 amino acids corresponding to a portion of a TCR chain which are immunogenic, that is, capable of inducing an immune response when injected into a subject, or a functional derivative of the peptide. The TCR is one which is utilized by T cells responsible for the specific allograft reaction, as described herein, or by antigen-specific T cells responsible for the specific contact sensitivity reaction, or involved in the allergen-specific antibody response, as described herein.

It is understood that the amino acid sequence comprising the TCR peptide of this invention can be used alone or bound to, or contained within the sequence of, a longer peptide. The longer peptide may carry additional sequence derived from the TCR of interest or may include sequences of an unrelated peptide, such as a carrier protein used to enhance the immunogenicity of the TCR oligopeptide.

In general, the peptide sequence represents a portion of the TCR itself and preferably corresponds to a portion of the TCR which is extracellular, exposed to antibody or other T cells, and is of biological importance in the activity of the T cell bearing the TCR. For the purposes of this invention, the peptide must be immunogenic, as defined below.

Peptides of the invention include those corresponding to a portion of the V region of the TCR. More preferably, the peptide corresponds to a segment of the VDJ region of the TCR β chain or the VJ region of the TCR α chain. In a preferred embodiment, the peptide corresponds to at least part of one of the three complementarity determining regions (CDRs) of the TCR heterodimer, such as second CDR (CDR2). Also intended within the scope of this invention are peptides corresponding to at least part of the TCR gamma and delta chains, their V regions, and CDR structures or their homologs (see Strominger, J. L., Cell **57**: 895-898 (1989): and Clevers, H. et al., Ann Rev. Immunol. **6**:629-662 (1988)).

The CDRs of the TCR are defined by analogy to the structure of the immunoglobulin molecule wherein the CDRs comprise the amino acid sequences of the heavy or light chain variable regions which contact antigen and constitute crucial portions of the antigen-binding site. All three TCR CDRs are believed to participate in binding to antigen and MHC (Davis, M.M., et al., Nature **334**:395-402 (1988): Claverie, J.M., et al., Immunol. Today **10**:10-14 (1989)). By directing the immune response of the subject, the protective antibodies or the protective T cells generated by TCR peptide vaccination against one of the CDRs of the target TCR, the likelihood of disrupting necessary binding or recognition events between the T cell involved in the graft rejection response and the antigen and/or MHC, or between the T cell involved in allergy or hypersensitivity and the antigen or hapten and/or MHC, is increased.

Functional derivatives of the peptides of the present invention include "fragments " "variants " "analogs" and "chemical derivatives". A "fragment" of the peptide of the present invention refers to any subset of the molecule, that is, a shorter peptide. A "variant" of the peptide refers to a molecule substantially similar to either the entire peptide or a fragment thereof. An "analog" of a peptide refers to a non-natural molecule substantially similar to either the entire molecule or a fragment thereof. A "chemical derivative" of a peptide of this invention contains additional chemical moieties not normally a part of the peptide.

For preparation of TCR peptides, the T cells recognizing an allogeneic antigen are obtained from the prospective recipient of an allograft. The naive T cells may be activated **in vitro** in a mixed lymphocyte culture reaction. T cells may also be obtained from a person who has received an allograft.

In another embodiment, the antigen-specific T cells recognizing an allergen, reactive hapten, or other antigen associated with contact sensitivity are isolated from a subject who is susceptible to or undergoing an allergic or contact or sensitivity response. The cells thus obtained may be expanded in culture. Additionally, they may be cloned, and if desired, may be fused to an immortalizing cell, such as a long term T cell line, a T cell lymphoma line or a T cell hybridoma, and grown in culture. The cultured cells serve as the source of cDNA encoding the appropriate TCR. Such cDNA is cloned and expressed by methods well known in the art. (See, for example, Sambrook, J. et al., Molecular Cloning: A Laboratory Manual, (1989)).

In one embodiment of this method, a subject's peripheral blood lymphocytes are cultured with the allogeneic antigen or with the specific antigen for several days, for example for 5-6 days. In other embodiments, cells are stimulated for longer periods of time. The time required for stimulation is a function of the proportion of reactive cells in the blood sample, the activation state of these cells, and the potency of the stimulating preparation, and is readily determinable by one of skill in the art. After culture under such selective conditions, viable cells are isolated and restimulated with autologous antigen-presenting cells (irradiated to prevent their proliferation, such as with about 2000-5000 rad) and a stimulatory concentration of the antigen, readily determined by one of skill in the art. About 7 days later, viable cells are collected and cloned by limiting dilution in the presence of antigen presenting cells and interleukin 2 (1L-2) or crude or pure combinations of additional lymphocyte growth factors (such as, for example, 1L-4). Such T cells are expanded and grown as a line and can be multiply restimulated with antigen-presenting cells, antigen preparations, and 1L-2. Restimulation can typically be carried out once a week. If desired, such T cells can be cloned by any of a number of methods known in the art, such as, for example, limiting dilution or by picking cells from colonies growing in soft agar, generally about 2 days after restimulation.

The present invention is further directed to pharmaceutical compositions useful for preventing or treating graft rejection or allergic reactions which comprise a vaccine of whole appropriate T cells treated in accordance with the present invention or membrane preparations or TCR proteins or peptides thereof and a pharmaceutically acceptable carrier or adjuvant.

The vaccines can be administered at approximately two to six week intervals, preferably monthly, for a period of from one to four inoculations in order to provide protection against allergic reactions. Each inoculation comprises between about $10^6$ and about $10^8$ cells, preferably between about $10^7$ and $10^8$ cells, or an equivalent amount of a membrane preparation. Alternatively, low doses of cells ("subpathogenic" doses as described by Lider et al., supra) in the range of about $10^3$ to about $10^5$ cells, may also be used.

Compositions within the scope of the invention include compositions wherein the active ingredient is contained in an effective amount to prevent development or exacerbation of graft rejection, or to prevent development of allergic reactions upon exposure to the particular allergen. Determination of the effective amounts can readily be made empirically by those of ordinary skill in the art without undue experimentation.

The compositions of the present invention can be administered by any of a number of means and routes known in the art. For example, administration may be by parenteral, subcutaneous, intravenous, intramuscular, intraperitoneal, transdermal or oral routes. The dosage administered will be dependent upon the age, health, and weight of the recipient, kind of concurrent treatment, if any, frequency of treatment, and the nature of the effect desired. The preferred route of administration for a whole T cell vaccine is the subcutaneous route.

T cell vaccines prepared as described above are administered to a subject who is receiving a graft at a dose range of about $10^6$ to $10^8$ cells, preferably about $10^7$ to $10^8$ cells, or an equivalent amount of a membrane preparation, on one or more occasions before the transplant. Treatment may also be continued into the post-transplant period, as can be determined by one of skill in the art without undue experimentation. Alternatively, low doses of cells ("subpathogenic" doses as described by Lider et al., supra) in the range of about $10^3$ to about $10^5$ cells, may also be used.

Effective doses of the peptides of this invention for use in preventing, or suppressing graft rejection are in the range of about 1 ng to 100 mg/kg body weight. A preferred dose range is between about 10 ng and 10 mg/kg. A more preferred dose range is between about 100 ng and 1 mg/kg. The immunogenicity of the peptide may be enhanced by including it in a longer peptide or chain or by conjugating it to "immunological" carriers, such as KLH, serum albumin, tetanus toxoid, and the like, using standard linking techniques. A variety of such methods is known in the art, e.g., use of condensing agents such as dicyclohexylcarbodiimide or use of linkers, such as those commercially available from Pierce Chemical Co., Rockford, IL.

In addition to the cross-linked T lymphocytes or membrane preparations or TCR proteins or peptides thereof which themselves are pharmacologically active, the pharmaceutical compositions may contain suitable pharmaceutically acceptable carriers comprising excipients and auxiliaries which facilitate processing of the active compounds into preparations which can be used pharmaceutically. Preferred compositions include the inclusion of an adjuvant, such as alum, or other adjuvants known in the art. (See, for example, Warren, H.S. et al., Ann. Rev. Immunol. **4**:369-388 (1986); Chedid, L., Feder. Proc. **45**:2531-2560 (1986)). Preferred carriers for the T cell vaccine compositions of the present invention are 0.01-0.1M, preferably 0.05M, phosphate buffer, or 0.8% saline.

Other pharmaceutically acceptable carriers for the compounds according to the present invention are liposomes, pharmaceutical compositions in which the active ingredient is contained either dispersed or variously present in corpuscles consisting of aqueous concentric layers adherent to lipidic layers. The active ingredient may be present both in the aqueous layer and in the lipidic layer, inside or outside, or, in any event, in the non-homogeneous system generally known as a liposomic suspension.

The compositions, methods, and products of this invention are applicable to human and veterinary uses. The preferred subject is a human.

The following examples are intended to be illustrative of, but not to limit, the invention.

EXAMPLE I

Sensitization and Elicitation of Contact Sensitivity

BALB/c female mice purchased from the Jackson Laboratories (Bar Harbor, ME) were used at 8-12 weeks of age. Groups of at least four mice were sensitized on the shave abdominal skin with either 25 $\mu$l of 0.5% 2,4-dinitro-1-fluorobenzene (DNFB) or 25 $\mu$l of 2% 4-ethoxymethylene-2-phenyl oxazolone (Ox) in a vehicle of 4:1 acetone:olive oil (by volume). Sensitization with picryl chloride (PiCl; 2,4,6-trinitrochlorobenzene) was performed with 75 $\mu$l of a 5% solution in the same diluent. Double sensitization with DNFB and Ox was accomplished by painting the right side of the abdomen with Ox and the left side of the abdomen with DNFB, leaving a strip of unshaven abdomen in the center.

DNFB and DNBS (2,4-dinitrobenzene sulfonic acid sodium salt) were obtained from Eastman-Kodak Co. (Rochester, NY) and Ox was obtained from BDH Chemicals, Ltd. (Poole, United Kingdom). Picryl chloride and glutaraldehyde were obtained from Fluka (Buchs, Switzerland).

For elicitation, mice were challenged by painting the ear pinna with either 20 $\mu$l of 0.2% DNFB or 20 $\mu$l of 0.5% Ox (10 $\mu$l to each side of the ear pinna) in 4:1 acetone:olive oil (by volume). Elicitation by PiCl was performed with 10 $\mu$l of 1% solution, in olive oil (5 $\mu$l to either side of the ear pinna). A constant area on the ears was measured immediately before challenge and 24 hours after challenge with a Mitutoyo dial thickness gauge micrometer. The contact sensitivity reaction was determined as the amount of ear swelling which is the difference between the two measurements expressed as mean units of ear swelling ($10^{-4}$ inch or $10^{-2}$ mm, depending on the micrometer used) $\pm$ the standard error (SE) of the mean. Inhibition of ear swelling was calculated as:

$$\text{\% Inhibition} = \frac{\text{positive control} - \text{experimental group}}{\text{positive control}} \times 100$$

In all calculations of inhibition, background ear swelling (the irritation effect) caused by DNFB or Ox in unsensitized mice was subtracted from the other groups. Student's t-test (two tailed) was used to determine the statistical significance between experimental and control groups.

EXAMPLE II

Transfer of Contact Sensitivity

DNFB-immune lymph node cells [I-LNC (DNFB)] or Ox-immune lymph node cells [I-LNC (Ox)] were obtained from donor mice sensitized on Day O and 1 with a total of 55 $\mu$l of 0.5% DNFB in 4:1 acetone:olive oil (25 $\mu$l applied to the shaved abdomen and 5 $\mu$l to each of the feet and ears). On day 4 (for DNFB) (or Day 5 for Ox) the draining lymph nodes were removed and single cell suspensions were prepared by pressing the lymph nodes through stainless steel screens. The cells were washed twice and resuspended in RPMI 1640 medium.

Recipients of immune lymph node cells were injected with 5 x $10^7$ I-LNC intravenously. Recipients were ear challenged within one hour of transfer. In co-transfer experiments, I-LNC from two different donor groups were mixed (1:1) and injected intravenously into the same mouse.

EXAMPLE III

Preparation of T Lymphocyte Vaccines and Vaccination

I-LNC obtained from donor mice as described above were resuspended (3-5 x $10^6$ cells/ml in Dulbecco's modified Eagle's medium, supplemented with glutamine (1 mM), 2-mercaptoethanol (5 x $10^{-5}$ M) and 1% autologous mouse serum. Concanavalin A (Yeda, Rehovot, ISRAEL), at a final concentration of 1.2 $\mu$g/ml, was added to activate the T lymphocytes. After 48 hours of incubation at 37°C under 5% $CO_2$, the activated cells were washed twice with phosphate buffered saline (PBS), pelleted, and resuspended in 3 ml PBS ($10^8$ cells). An equal volume of 0.6% glutaraldehyde (from Fluka AG, Buchs, Switzerland) (w/v) in PBS was then added and incubated at room temperature for 15 minutes. The treated I-LNC were washed 4-5 times by centrifugation in 50 ml of PBS.

Vaccination was performed by intraperitoneal (IP) injection of the treated cells into recipient mice (2-3 x $10^7$ cells/0.5 ml per mouse).

EXAMPLE IV

Lymphocyte Responses to Antigen In Vitro

Lymphocytes from draining lymph nodes were prepared as described above. Then, 1 x $10^5$ viable cells were added to round-bottomed, 96-well culture plates containing 0.2 ml RPMI-1640 supplemented with 0.3 mg/ml L-glutamine, 100 U/ml penicillin, 100 $\mu$g/ml streptomycin, and 5% heat inactivated fetal calf serum. The cells were cultured for 72 hours at 37°C in a humidified atmosphere of 5% $CO_2$ in the presence or absence of 2,4-dinitrobenzene sulfonic acid sodium salt ($DNBSO_3$, from Eastman-Kodak) as the specific antigen. This antigen has the same dinitrophenyl haptenic moiety to which cells from DNFB-sensitized subjects are specific. Cultures were pulsed with 1 $\mu$Ci of [$^3$H]-thymidine for the last 18 hours of incubation before harvesting with an automated sample harvester. The incorporation of the isotope into cells in triplicate cultures is expressed in one of two ways: Net cpm ± SE; or

$$\text{Stimulation Index (SI)} = \frac{\text{cpm of cells} + \text{DNBSO}_3}{\text{cpm of cells} - \text{DNBSO}_3}$$

Student's t-test (two tailed) was used to determine the statistical significance between experimental and control groups.

EXAMPLE V

Induction of Tolerance by T Cell Vaccine

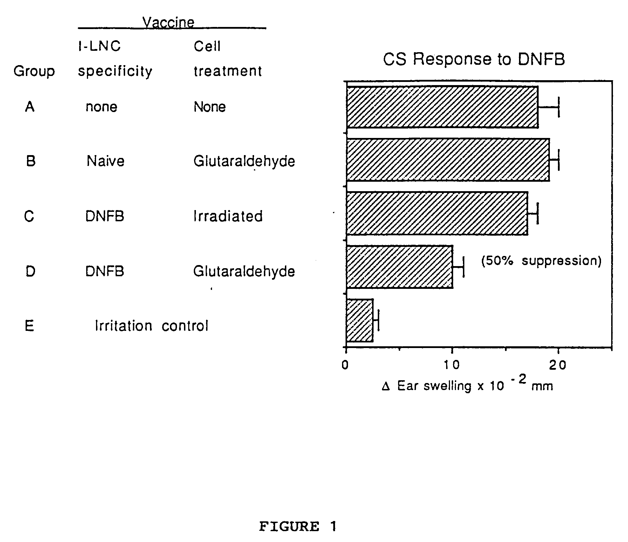

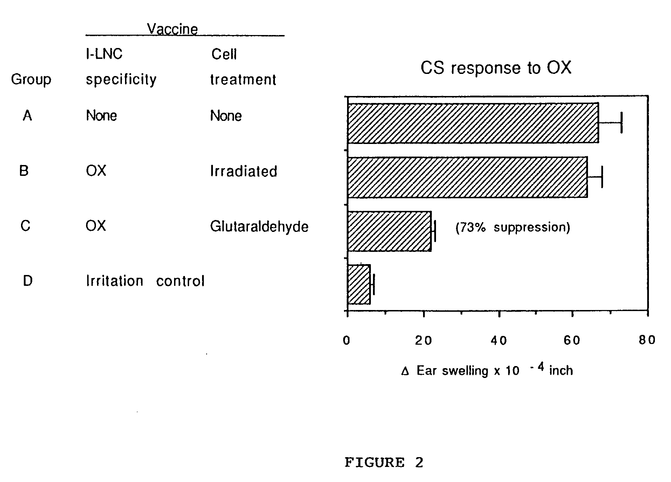

Figures 1 and 2 summarize the results of experiments testing the effect of vaccination of mice with glutaraldehyde treated I-LNC on the development of actively induced contact sensitivity to two antigens, DNFB and Ox.

Vaccinating mice with glutaraldehyde-treated I-LNC(DNFB) 7 days prior to sensitization with DNFB or Ox caused 50% suppression of the response to DNFB as compared to that of unvaccinated mice (Figure 1, group D vs. A, p< 0.002). Similarly, 73% suppression of the contact sensitivity response to OX was caused by vaccination with glutaraldehyde treated I-LNC (Ox) (Figure 2, group C vs. A, p<0.001).

Two different control groups were included in these experiments: (1) mice that had been vaccinated with gamma-irradiated I-LNC (Figure 1, group C and Figure 2, group B); and (2) mice that had been vaccinated with glutaraldehyde-treated naive (i.e. non-immune) lymph node cells (N-LNC) (Figure 1, group B).

Irradiation destroyed the ability of the I-LNC to suppress immunity. Similarly, non-immune LNC were unable to vaccinate against contact sensitivity.

Thus, a marked state of tolerance to contact sensitivity could be achieved by vaccination with glutaraldehyde treated I-LNC. This tolerance could not be attributed to antigen carry-over, since irradiated I-LNC, which presumably would carry over similar amounts of antigen, were inactive.

As in other forms of T cell-mediated immunity, T lymphocytes from mice contact sensitized to DNFB show antigen-specific T cell proliferative responses in vitro. Therefore, tests were conducted to determine if the tolerance to DNFB induced by T cell vaccination affected in vitro proliferative responses to the hapten. I-LNC from normally sensitized mice showed significant proliferative response to DNBSO_3 (stimulation indices of about 5). I-LNC from mice that had been vaccinated with irradiated I-LNC (DNFB) showed similar proliferative responses. However, when mice had been vaccinated with glutaraldehyde-treated I-LNC-(DNFB), their T cell proliferative response to DNBSO_3 was significantly reduced (p<0.01).

EXAMPLE VI

Immunological Specificity of Vaccination

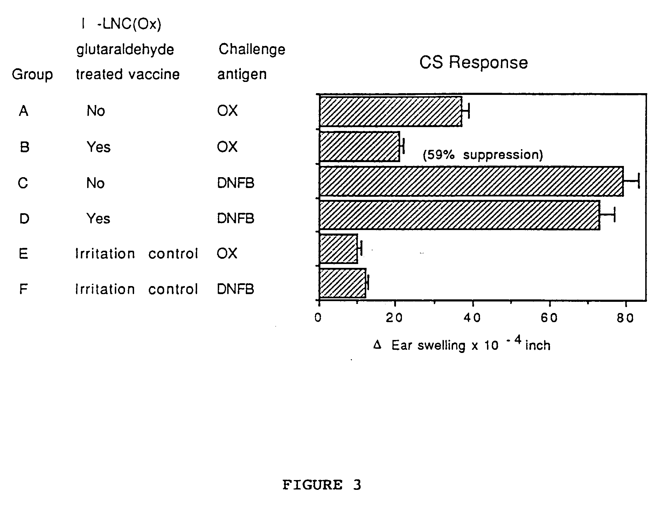

Two types of experiments were conducted to test the antigen specificity of T cell vaccination against contact sensitivity. First, mice were vaccinated with glutaraldehyde treated I-LNC (Ox), and seven days later were with a combination of Ox and DNFB. Five days after sensitization with both antigens, individual responses were evoked by challenge with either DNFB or Ox. As shown in Figure 3, in mice vaccinated with glutaraldehyde-treated I-LNC(Ox) and later sensitized with both Ox and DNFB, there was significant inhibition (50%) of the response to Ox (group B vs. A, p<0.002). The contact sensitivity response to DNFB was unaltered (group C vs. D, not significant). As above, irradiated I-LNC failed to vaccinated against contact sensitivity.

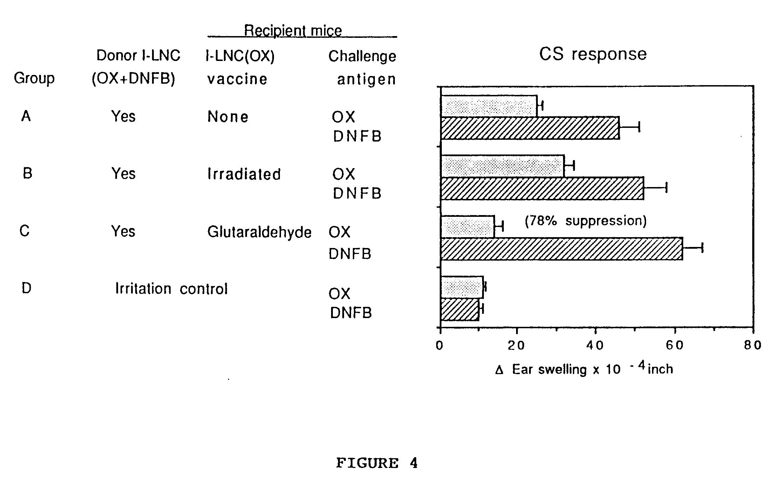

The second set of experiments, of which a representative is shown in Figure 4, utilized an adoptive transfer approach. Here, the ability to transfer a state of immunity to a naive recipient was tested in normal or vaccinated recipients. Specifically, a mixture of I-LNC(DNFB) and I-LNC(Ox) were transferred together into recipient mice that had been vaccinated a week earlier with glutaraldehyde treated I-LNC(OX) (group C), or left unvaccinated (group A). The left ears of the recipients were then challenged with DNFB and the right ears with Ox.

In mice vaccinated with I-LNC(Ox), potent suppression (78%) of the response to Ox was observed, whereas the response to DNFB was unaffected (groups C and A). If the vaccinating cells had been irradiated (group B), there was no effect of vaccination; these recipients responded just as did unvaccinated mice (group A).

Thus, T cell vaccination resulted in immunologically specific inhibition of the state of contact sensitivity induced either by antigen or by passive transfer of immune lymphocytes.

## EXAMPLE VII

### Anti-idiotypic DTH to the T Cell Vaccine

Since, the antigen-specificity of the vaccinating T cells influenced the antigen specificity of the tolerance produced, the possibility that vaccination involved the induction of an anti-idiotypic response to the T cell receptors (TCR)s of the vaccine T cells was tested.

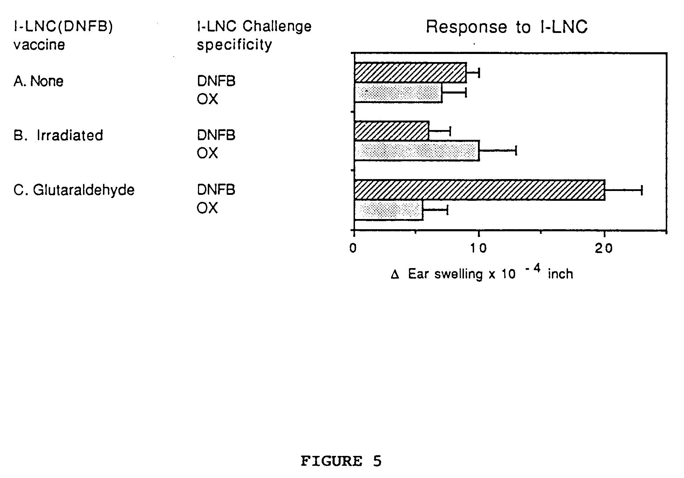

For this purpose, the presence of a delayed type hypersensitivity (DTH) response of vaccinated mice to the specific I-LNC was investigated (Figure 5). Mice were vaccinated with glutaraldehyde-treated I-LNC-(DNFB) (Group C) or left unvaccinated (Group A), and sensitized with DNFB 7 days later. Five days after sensitization, DTH responses were evoked by intradermal injection into the left ear pinna of vaccine-type T cells, I-LNC(DNFB), which had activated in vitro with concanavalin A and irradiated (2500R). Right ears were injected with control T cells, I-LNC(Ox) which had been similarly treated. Ear swelling responses were measured 24 hours later.

Non-vaccinated control mice (group A) showed background level ear swelling to both I-LNC (Ox) and I-LNC (DNFB). Mice vaccinated with irradiated I-LNC(DNFB) (group B), showed similar responses, indicating no effect of vaccination. In contrast, mice given the competent vaccine, glutaraldehyde treated I-LNC-(DNFB) (group C), which had already been shown to induce a state of resistance to antigen-specific contact sensitivity, manifested a DTH response to I-LNC(DNFB). This responses was "vaccine-specific," since no DTH reactivity above background to I-LNC(Ox) was observed in the contralateral ear.

It was thus concluded that the tolerance to contact sensitivity produced by vaccinating mice with immune T cells is associated with a T cell response specific for the vaccinating T cells.

These findings extend the concept of T cell vaccination beyond the area of autoimmune diseases, to include contact sensitivity to defined chemical allergens.

In terms of mechanistic explanation, it may be said that T cell vaccination is effective in autoimmunity because anti-idiotypic networks arise naturally during development of the immune system as a safeguard against autoimmune disease. Thus, vaccination using T cells reactive to self-antigens might exploit the pre-existing network and even strengthen it.

The results reported herein indicate further that T cell vaccination may lead to tolerogenesis for simple chemical structures, such as the haptens of DNFB and Ox.

## EXAMPLE VIII

### T Cell Vaccination Against IgE Responses

Experiments were performed to test whether T cell vaccines described above were useful in inhibiting IgE production, which is central in immediate hypersensitivity responses.

T cell vaccines consisted of Concanavalin A activated, glutaraldehyde-treated I-LNC(DNFB) described above. These cells are specific for the dinitrophenyl (DNP) hapten structure. They were injected into recipient mice IP as described above.

DNP-specific IgE antibody responses were induced using the method of Levine and Vaz (Int'l. Arch. Allergy Appl. Immunol. 39: 156 (1970)). Mice received an IP injection of 100 $\mu$g DNP-BSA (DNP-coupled bovine serum albumin) in 10 mg of $Al(OH)_3$.

Control BALB/c mice (5) in group A were immunized with DNP-BSA as above on days 8 and 22. Five experimental mice (group B) were vaccinated with the T cell vaccine on days 0, 6 and 15, and received DNP-BSA on days 8 and 22. Sera was obtained on days 0 (pre-immune), 22, and 27.

Anti-DNP antibodies of the IgE isotype were measured using an enzyme immunoassay (EIA) as described by Hirano et al. (J. Immunol. Meth. 119:145 (1989)) with minor modifications. Microtiter plates were coated with DNP-coupled keyhole limpet hemocyanin (DNP-KLH) at 2 $\mu$g/ml. The plates were blocked with 1% (w/v) casein. Sera being tested or a positive control reagent were added at several dilutions. The positive control was a purified monoclonal antibody of the IgE isotype specific for DNP (SPE-7) (Eshhar, Z. et al., J. Immunol. 124:775 (1980)). Rat antibodies specific for murine IgE were added. Then, peroxidase-conjugated mouse anti-rat IgG antibodies were added to wells. The ELISA was developed using a chromogenic substrate for peroxidase, o-phenylenediamine (OPD), 0.8%. The colored reaction product was measured using an EIA plate reader using an absorption value of 492 nm.

The results are shown in Table 1, below.

TABLE 1

| Antibody Titer (Absorption units) on DAY: | | | |
|---|---|---|---|
| Group | 0 | 22 | 27 |
| A | 0.22 | 1.35 | 0.28 |
| B | 0.24 | 0.41 | 0.16 |
| % Suppression | 0 | 70 | 43 |

These results indicate that an antigen-specific IgE is significantly suppressed by vaccination with antigen-specific T lymphocytes.

## EXAMPLE IX

### INHIBITION OF THE MIXED LYMPHOCYTE REACTION BY T CELL VACCINATION

The present study was done to examine the effect of T cell vaccination on the ability of T cells from vaccinated rats or mice to react in a one-way mixed lymphocyte reaction (MLR) **in vitro**. The MLR has long been known in the art to serve as an **in vitro** correlate of the **in vivo** allograft reaction.

### MATERIALS AND METHODS

#### Animals

Four to 6 week-old female mice and 6-8 week old female rats were used. BALB/c (H-$2^d$), C3H/eB (H-$2^k$) and C57BL/6 (H-$2$) mice were purchased from Jackson Laboratories (Bar Harbor, Maine). Inbred Lewis (RTI$^1$), Brown-Norway (BN) (RT1$^n$) and Wistar-Furth (Wistar) (RTI$^u$) rats were obtained from the Experimental Animal Breeding Center of the Weizmann Institute of Science.

#### T cell vaccination

BALB/c mice were primed by injecting 5 x $10^6$ C57BL/6 splenocytes into each hind footpad. Draining popliteal and inguinal lymph nodes (LN) were excised 1 week later and the lymphocytes stimulated by culture for 48 h with 1.25 $\mu$g/ml Con A in Dulbecco's modified Eagle's medium, containing antibiotics, 2-mercaptoethanol and 1% normal mouse serum (Ben-Nun et al., Eur.J.Immunol., **11**:195 (1981)). LN from naive BALB/c mice activated with Con A in the same manner served as control vaccines.

The inventors and their collaborators previously showed (Mor et al., J.Clin.Invest., **85**:1594 (1990)) that activation of T cells **in vitro** from rats primed **in vivo** with specific antigens selects for antigen-specific memory cells and markedly amplifies the T cell responses to the specific antigen and to alloantigens in an MLR.

For vaccination, the BALB/c anti-C57BL/6 or naive activated lymphocytes were cross-linked by a 15 min incubation with 0.3% glutaraldehyde (Lider et al., Proc. Natl.Acad.Sci. U.S.A., **84**:2577 (1987)) and then washed thrice in phosphate buffered saline (PBS). Naive BALB/c mice were treated with 2 x $10^7$ vaccinating cells injected intraperitoneally and the vaccination was repeated one and two weeks later. An MLR was performed a week after the third vaccination.

A similar protocol was used in rats. Lewis rats were primed with 5 x $10^6$ BN or Wistar splenocytes in each hind footpad, and popliteal and inguinal LNs were removed one week later. The Lewis LN cells containing anti-BN or anti-Wistar T cells were stimulated with Con A **in vitro** and used as T cell vaccines to vaccinate Lewis rats.

To induce an anti-ergotypic response, Lewis rats were inoculated with Lewis spleen cells that had been activated with Con A (Lohse et al., supra) and treated with glutaraldehyde (Lider et al., 1987, supra).

### Mixed Lymphocyte Reactions

MLR cultures were done in 96-well round-bottom plates. Responder cells (2.5 x $10^5$) were seeded in each well and 5 x $10^5$ irradiated (1500 R) stimulator cells were added. The culture medium used was as above. Cells were cultured for 96 h and labeled with 1 $\mu$Ci $^3$H-thymidine per well for the last 18 h of culture. Cultures were harvested and radioactivity determined in a liquid scintillation counter.

The MLR of either spleen or LN lymphocytes was measured. Vaccinated animals were tested one week after the last vaccination. Control animals were always of the same age as experimental animals.

### Active Suppression

Active suppression by cells from vaccinated animals was tested using 2.5 x $10^5$ responder cells from naive control animals and 2.5 x $10^5$ responder cells from vaccinated animals, seeded together in the same well with 5 x $10^5$ irradiated stimulator cells. In order to exclude cell crowding as a cause of a decreased MLR, two types of control experiments were included. First, 5 x $10^5$ responder cells from control animals, seeded together with stimulator cells resulted in an MLR almost identical to that obtained with 2.5 x $10^5$ responder cells. In addition, suppression was also obtained using 1.25 x $10^5$ responder cells.

### RESULTS

### T cell vaccination induces depression of the MLR.

Figure 6a shows that vaccination of Lewis rats with Lewis anti-BN LN cells significantly reduced the MLR of Lewis rats against BN cells from 68,000 cpm to 21,000 cpm. Moreover, the MLR of Lewis against Wistar cells was similarly reduced by vaccination of the Lewis rats with Lewis anti-BN LN cells (Figure 6a). A similar but less marked reduction in MLR was obtained after vaccination with splenocytes from Lewis rats primed with BN cells (Figure 6b). Thus, vaccination of Lewis rats with activated Lewis anti-BN lymphoid cells could induce a pronounced decrease in the MLR, which did not seem to be MHC-specific. A non-MHC specific reduction in MLR was also achieved by vaccination with Wistar-primed LN cells and splenocytes (Figure 6c).

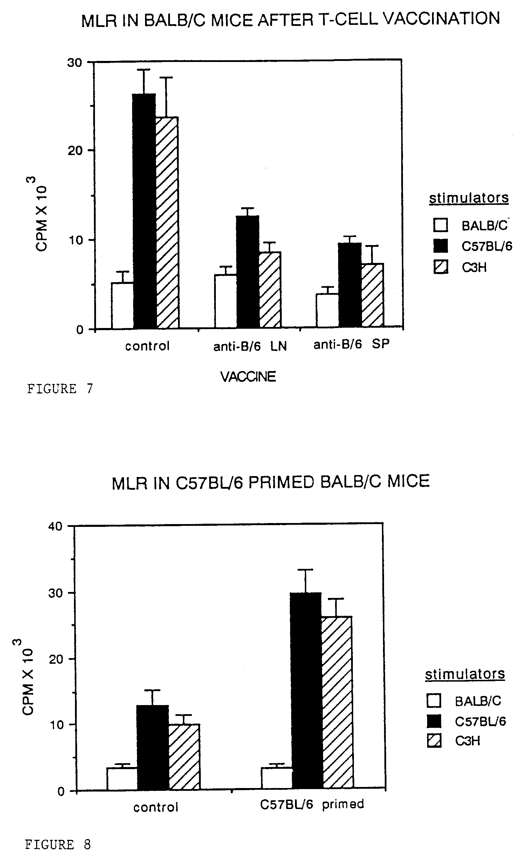

Comparable results were obtained in experiments in mice. BALB/c mice vaccinated with LN cells or splenocytes from BALB/c mice sensitized to C57BL/6 mouse cells showed a marked decrease in their MLR, both against C57BL/6 and against C3H/eB splenocytes (Figure 7). Thus, both in rats and in mice, alloreactivity against diverse MHC antigen-bearing stimulator cells could be markedly reduced by vaccination with lymphocytes primed with allogeneic cells of one MHC haplotype.

### Non-MHC specific augmentation of the MLR by priming in vivo.

The above findings suggested that the allogeneically primed T cells used for T cell vaccination might not be MHC-specific. This interpretation is supported by the observation that the MLR against different allogeneic stimulator cells was actually increased by immunization with cells from just one allogeneic strain. Figure 8 shows that after priming BALB/c mice with irradiated splenocytes from C57BL/6 animals, the MLR of cells obtained from the recipients was markedly increased, and this increase was of the same magnitude when tested against C57BL/6 stimulator cells as when tested against C3H/eB stimulator cells. Thus, the apparent lack of MHC specificity in the reduction of the MLR induced by T cell vaccination was associated with a similar lack of MHC specificity in the T cells used for vaccination. In other words, the population of lymphoid cells whose MLR was nonspecifically augmented by priming with allogeneic cells was used as the T cell vaccine, and vaccination with these T cells induced depression of the MLR that also was not restricted by MHC alleles.

### MLR depression is not induced by an anti-ergotypic response

Since an anti-ergotypic response, previously shown to be unrestricted by MHC alleles (Lohse et al., supra) may be involved, experiments were conducted to test this possibility. Accordingly, Lewis rats were vaccinated with Lewis T cells activated **in vitro** with Con A. Immunization with such syngeneic activated T cells induces an anti-ergotypic response (Lohse et al.,supra).

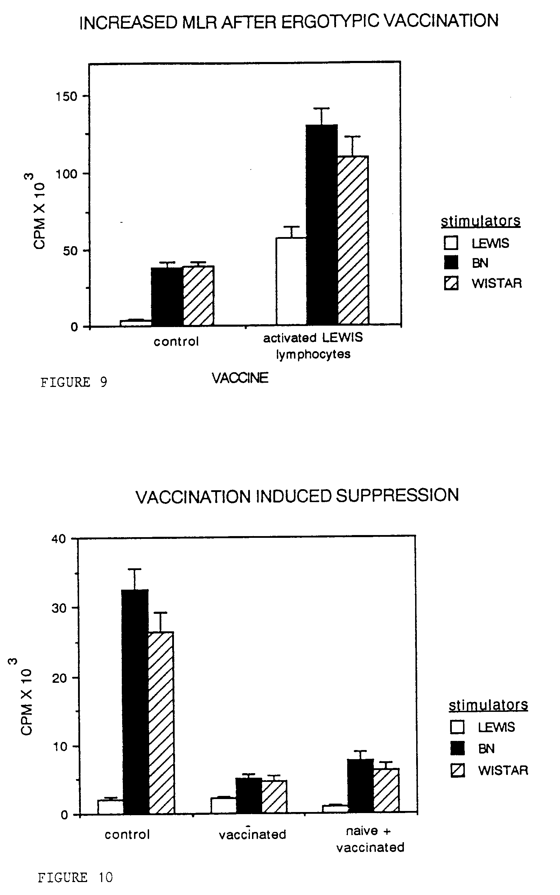

Figure 9 shows that the cells of ergotypically primed Lewis rats actually augmented the MLR to allogeneic stimulator cells **in vitro**, as it augmented the proliferative response to activated syngeneic stimulator cells. Thus, the reduction of the allogeneic MLR seen after T cell vaccination with alloantigen-primed cells was not likely to be due to an anti-ergotypic response. Similar amplification of the MLR was obtained when an antigen-activated mycobacteria-specific clone (A2b) was used to prime an anti-ergotypic response.

## Active suppression

Experiments were done to test whether the reduced MLR after T cell vaccination was due to deletion of MLR reactive lymphocytes or to their active suppression. The results summarized in Figure 10 suggest that suppression played an important role: adding lymphocytes from vaccinated animals to responding lymphocytes from naive animals led to a marked suppression of the MLR. Thus, the response of naive lymphocytes was inhibited by the presence of lymphocytes from vaccinated animals.

## DISCUSSION

The results described above show the effect of T cell vaccination on reducing alloantigen responsiveness in the MLR, both in rats and mice. Unexpectedly, the reduction of the MLR was not restricted to the MHC alleles used to prime the animals from which the T cell vaccine was prepared. However, the cells used to prepare the vaccine also showed no MHC allelic restriction in their MLR response after priming with allogeneic cells. Thus, the T cells used for vaccination were themselves not MHC restricted, despite coming from donors primed with MHC specific allogeneic stimulator cells **in vivo**. The depression in MLR following T cell vaccination may only reflect the lack of specificity demonstrated by the augmented reactivity of the T cell vaccine itself. Alternatively, the MHC-unrestricted amplification of the response of the vaccinating cells may be due to some general effect of priming, such as increased responsiveness to, or production of, lymphokines, and may not be causally related to the depressed MLR induced by vaccination.

Active suppression of responding T cells in the vaccinated animals seemed to account for the depression of the MLR. Induction of an anti-ergotypic response with naive activated, syngeneic cells, had the opposite effect, causing augmentation of the MLR. This indicates that the lack of MHC allelic restriction cannot be due to an anti-ergotypic response alone.

Binz, Wigzell and their colleagues described reduced MLR and prolonged skin graft survival after immunization of rats with anti-MHC antibodies in complete Freund's adjuvant (Binz et al., Nature, **262**:294 (1976); J.Exp.Med., **144**:1438 (1976)). In these reports, Lewis rats immunized with anti-DA antibodies showed a depressed response to DA cells but a normal response to third party BN cells. It was not clear whether this effect could also be achieved in other animal species and in other MHC combinations. The induction of anti-idiotypic antibodies including anti-TCR antibodies was postulated to underlie this phenomenon. The difference in results presented herein from those of Binz et al. may be due to the difference in "vaccination" protocol. That is, in the present invention, the animals were vaccinated with syngeneic, activated T cells from alloantigen-primed animals rather than with specific anti-MHC antibodies in adjuvant. These dissimilar forms of induction may certainly have activated different regulatory mechanisms leading to diverse forms of MLR depression.

There is evidence that the specific effects of T cell vaccination, those not due to the non-specific anti-ergotypic response, are determined by the specificity of the TCRs of the vaccinating cells. For example, T cells reactive to different epitopes on myelin basic protein were found not to cross-vaccinate against experimental autoimmune encephalomyelitis (EAE) (Holoshitz et al., J.Immunol., **131**:2810 (1983)).

Recently, it was reported that vaccination against EAE was attainable by immunization with peptides constructed from the amino acids of the $\beta$ chain of the TCR type preferentially used for the response to myelin basic protein (Vandenbark et al., supra; Howell et al., supra). If, indeed, T cell vaccination induces TCR-specific T cells (Lider et al., 1989, supra; Sun et al., supra; Vandenbark et al., supra) the present observation of suppressed MLR which appears to be independent of the MHC alleles appears paradoxical. It should be noted that the Vandenbark et al. study of vaccination against EAE used a TCR peptide sequence common to all $V\beta 8.2$ molecules. Although not tested, this peptide should have induced suppression of all T cells expressing a TCR $V\beta 8.2$ encoded receptor, irrespective of their antigen specificity. According to this interpretation, vaccination with the TCR $V\beta 8.2$ peptide was successful in EAE because murine EAE is largely restricted to T cells expressing $V\beta 8.2$ (Acha-Orbea et al., Ann.Rev.Immunol., **7**:371 (1989)).

Selective intrathymic deletion of T cells bearing certain $V\beta$ TCRs has been demonstrated to be MHC determined (Marrack et al., Science, **238**:1073 (1987); Pullon et al., Nature, **335**:796 (1988)). These studies suggest $V\beta$ restriction of the alloresponse. Now, if the anti-BN and anti-Wistar T cells developing in Lewis rats were to be restricted in their expression of a limited array of TCR, in particular of the same $V\beta$ or $V\alpha$ families, it is conceivable that these T cells, despite their recognition of different MHC allelic products, possibly via their diverse TCR VDJ regions, nevertheless vaccinate against their common peptide segments. The responding T cells stimulated by the vaccine T cells, seeing the common TCR peptide, would then be able to suppress equally both anti-BN and anti-Wistar T cell populations. In this sense, a TCR peptide shared by a variety of anti-MHC receptors might serve as a common regulatory element or target.

## EXAMPLE X

### Inhibition of heart allograft rejection by T cell vaccination

Groups of 10-15 Wistar rats (MHC type: $RT1^u$) were engrafted with hearts from allogenic BN ($RT1^n$) rats. Using a standard procedure of orthotopic heart transplantation, the allogeneic heart was anastomosed to the abdominal aorta and inferior vena cava. Rejection was monitored by feeling the pulsations of the transplanted heart through the wall of the abdomen. Rejection was determined by the cessation of the pulsations. Heart transplants which failed for technical reasons stopped beating within 3-4 days.

Control, non-vaccinated rats showed a very sharp rejection beginning on day 7, and none survived beyond 11 days. In contrast, the groups of rats treated with T cell vaccination showed markedly prolonged allograft survival.

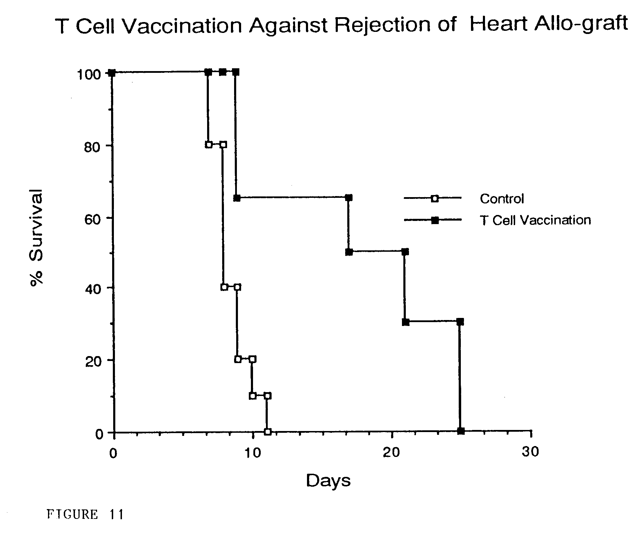

In a first experiment (Figure 11), Wistar rats were vaccinated with Wistar rat T cells that were selected for their proliferative responses to irradiated spleen cells of allogeneic BN rats. The vaccine was prepared by incubating Wistar rat spleen cells with an equal number of irradiated (2000 rad) spleen cells of BN rats to produce a standard MLR in which the Wistar T cells with receptors for BN histocompatibility antigens are specifically induced to proliferate. These activated Wistar-anti-BN T cells were then treated with glutaraldehyde (0.3%) for 3 minutes, as previously disclosed (see: Cohen, I.R. e tal. references, supra).

The test Wistar rats were engrafted with BN heart allografts and graft survival was determined. It can be seen that the survival time of the allografts in the vaccinated rats was extended up to 25 days.

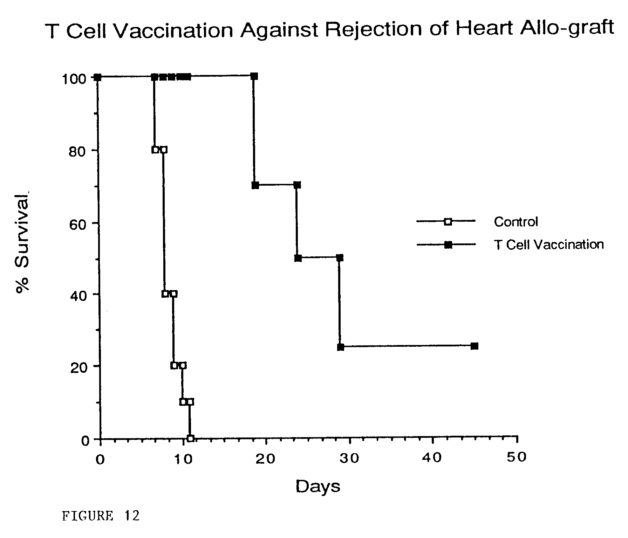

A second experiment was done in the same way except that the vaccine was prepared from Wistar T cells obtained from rats that had already rejected BN skin grafts applied 12 days earlier. In this protocol, the Wistar anti-BN T cells are considered to be more strongly sensitized due to the prior **in vivo** priming against BN histocompatibility antigens. These cells were then activated **in vitro** and treated with glutaraldehyde as above. Here too, there was a striking increase in graft survival (Figure 12): 20% of the rats maintained the BN heart graft for over 45 days.

## EXAMPLE XI

The aim of the present study was to examine the effect of T cell vaccination on an alloantigen response **in vivo**. The model of heterotopic cardiac transplantation in rats was used. Wistar rats were vaccinated with attenuated T cell vaccines from Wistar rats that had been sensitized against BN alloantigens. Control T cell vaccines were made from mitogen-activated Wistar T cells, or from Wistar T cells sensitized against third-party Hooded rats. The Wistar rats were then grafted with BN hearts.

## METHODS

**Animals:** Cardiac transplantation was performed between male inbred Wistar rats weighing 350-500 g serving as recipients, and male inbred Brown-Norway (BN) rats weighing 250-350 g serving as donors. Male inbred Hooded (Ho) rats were used to produce third party T cell vaccines. All rats were obtained from the Animal Breeding Center of the Weizmann Institute of Science.

**Skin Allografting:** Full thickness skin grafting was performed as previously described (Baharav, E. et al., (1986) J.Immunol.Methods **90**:143-144). One male rat served as a donor to up to 10 male recipients. Day of grafting was Day O. The day of rejection was defined as the day on which complete separation of the graft occurred.

**Cardiac Transplantation:** Heterotopic cardiac transplantation was performed under pentobarbital anaesthesia (50 mg/kg I.P.), using the modified technique of Ono and Lindsey (J.Thor.Cardiovas.Surg. (1969) **57**:225-229). Day of grafting was day O. Graft survival was assessed by daily palpation. The day of graft rejection was defined as the day of cessation of the beat of the cardiac allograft. Rejection was confirmed histologically. Technical failures within the first 72 hours were excluded from the experimental groups.

16

**T cell Vaccination :** Wistar rats were primed by transplanting BN or Ho skin grafts into the neck region. When the skin allografts were rejected, the draining cervical and axillary lymph nodes (LN) were excised and the lymphocytes were stimulated by culture for 48 h with 1.25 $\mu$g/ml Concavalin A (Con A) in medium containing antibiotics, 2-ME, and 1% autologous serum as described (Ben-Nun, A. et al., (1981) Eur.J.Immunol. 11:195-199). It was previously demonstrated that activation of T cells with Con A **in vitro** led to a substantial increase in the activity and frequency of T cells specific for antigens to which the rats had been sensitized **in vivo** (Mor, F. et al., (1990) J.Clin.Invest. **85**: 1594-1598). LN from naive Wistar rats activated with Con A in the same manner served as controls to differentiate between anti-ergotypic and antiidiotypic reponses (Lohse, A. et al., (1989) Science **244**: 820-822). The Wistar anti-BN, Wistar anti-Ho and Con A activated Wistar lymphocytes were attenuated by treatment for 15 min with 0.3% glutaraldehyde and then washed thrice in PBS. Wistar rats were vaccinated with three I.P injections of $2x10^7$ vaccinating cells, performed at days -14, -7 and +5 of cardiac transplantation. A fourth vaccination was done at day +12 when grafts survived that long.

In an alternative protocol, instead of skin allografting, Wistar rats were primed by I.P injection of $5x10^6$ BN splenocytes. Mesenteric, cervical and axillary (LN) were excised two weeks later and the vaccinating lymphocytes were prepared as described above.

**Statistics:** The Kaplan-Meier method was used to compute actuarial graft survival. The data obtained in each group were expressed as means±SEM, and the Mann-Whitney U test was used for calculation of intergroup significant differences.

## RESULTS

### Wistar, Brown-Norway and Hooded rats mutually rejected skin grafts

To establish the histocompatibility of the strains of rats used, skin grafting was performed. Table 2 shows that Wistar rats rejected BN skin grafts within 16.4±1.5 days and Ho skin grafts within 13.5±1.1 days. BN rejected Ho skin grafts within 13.7±0.1 days. Although it appears that BN skin grafts survived in W rats slightly longer than Ho grafts, the differences did not reach statistical significance.

Thus it may be concluded that each of the three strains differ substantially from the other two in their major histocompatibility loci.

Table 2.

| Survival of Wistar, BN and Ho skin allografts | | | | |
|---|---|---|---|---|
| Group | Recipient | Donor | Graft survival (days) | Mean±SEM |
| 1 | Wistar | BN | 9,13,13,15,15, 17,17,19,20,26 | 16.4±1.5 |
| 2 | Wistar | Ho | 10,11,11,11,12, 13,14,14,18,21 | 13.5±1.1 |
| 3 | BN | Ho | 10,12,14,14, 15,17 | 13.7±0.1 |

Wistar and BN rats received skin grafts from BN or Ho donors. Rejection was scored as the day of separation of the skin graft. Differences between the groups were not statistically significant.

### T cell vaccination prolongs cardiac allograft survival

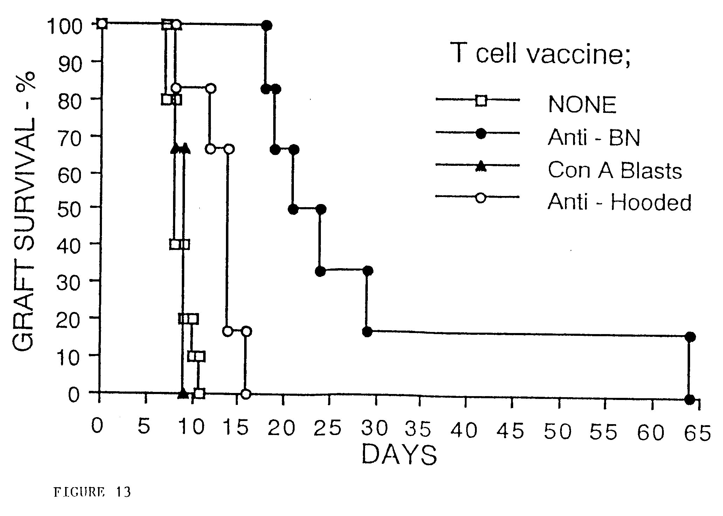

The results of heart transplantation are shown in Table 3 and Figure 13. Untreated W rats rejected BN cardiac allografts within 8.50±0.4 days (Group 1). Vaccination of those rats with Wistar anti-BN cells (Group 2) primed by skin allografts significantly prolonged cardiac allograft survival to 29.2±7.1 days (p<0.0001). Priming of the vaccine donor by BN splenocytes(Group 3) was less effective than priming by skin grafting: survival of cardiac allografts was 18.0±0.34 days; double that of the controls.

17

Table 3.   BN cardiac allograft survival in Wistar recipients treated with T-cell vaccination.

| Group | Wistar T-cell vaccine (Mode of priming) | Graft survival (days) | Mean±SEM | P values |
|-------|------------------------------------------|------------------------|----------|----------|
| 1 | None | 7,7,8,8,8, 8,9,9,10,11 | 8.5±0.4 | |
| 2 | Anti-BN | 18,19,21,24, 29,64 | 29.2±7.1 | <0.0001-vs.1 |
| 3 | Anti-BN | 9,17,21,25 | 18.0±3.4 | 0.025-vs.1 |
| 4 | Con A | 8,8,9,9,9,9, | 8.7±0.2 | NS-vs.1 |
| 5 | Anti-Ho | 8,12,14,14, 14,16 | 13.0±1.1 | 0.01-vs.1 0.001-vs.2 |

Wistar rats were grafted with BN heart allografts. Some of the rats were treated by a T cell vaccination with Con A activated Wistar T cell obtained from rats that were unprimed (group 4), or that had been primed with BN skin grafts (group 2), BN splenocytes (group 3), or Ho skin grafts (group 5). Rejection of the allograft was determined by cessation of the heart beat.

**Cardiac allograft survival is not influenced by an anti-ergotypic response**

As an anti-ergotypic control, Wistar rats were vaccinated with Con A activated, syngeneic T cells obtained from naive rats. It was previously shown that such a vaccination can successfully inhibit experimental autoimmune encephalomyelitis (EAE) in rats by induction of MHC-non-restricted anti-ergotypic response (Lohse A. et al. (1989), see above). In the present study this type of vaccination was followed by graft survival of 8.7±0.2 days, virtually identical to the control group (Table 3, Group 4, Figure 13). Thus, the prolongation of cardiac allograft survival achieved by T cell vaccination was not likely due to the induction of an anti-ergotypic response to activation markers on syngeneic T cells.

**The effect of T cell vaccination is not MHC-restricted**

To examine the antigen specificity of T cell vaccination, Wistar rats were vaccinated with Wistar anti-Hooded cells and then transplanted with BN cardiac allografts. Graft survival in this group (Group 5) was 13.1±0.1 days which, although much shorter than that achieved with the antigen-specific vaccine, was still significantly longer than the control groups (p = 0.01 group 5 vs. group 1 and p = 0.001 group 5 vs. group 2, Table 3, Figure 13).

Thus, it appears that T cell vaccination against the response to one strain of rats is able to induce some degree of tolerance to diverse allogeneic antigens.

**DISCUSSION**

The results of the experiments described here demonstrate that vaccination of rats with syngeneic T cells that have been primed against the donor MHC-antigens can induce tolerance manifested by a significant prolongation of cardiac allograft survival. This effect of T cell vaccination was most evident when the T cells had been obtained from rats specifically sensitized against the donor rats and it was achieved without detectable side effects. Similar to the effect of T cell vaccination on the MLR **in vitro**, the effect was non-MHC restricted. Vaccination of Wistar rats using anti-Ho T cells induced partial, but significant, tolerance to BN rat alloantigens. This lack of specificity cannot be attributed to an anti-ergotypic response induced by T cell vaccination, as vaccination of animals with naive, syngeneic, Con A activated T cells had no detectable effect on allograft survival.

Cross-reactivity between some histocompatibility antigens of BN and Ho rats could also account for partial inhibition of rejection of BN allografts by T cell vaccination with Wistar anti-Ho cells. Enhancement of survival of kidney allografts may be induced by pre-transplant blood transfusion using a third-party blood donor (Lagaaij, E.L. (1989) N.Eng.J.Med. **321**:701-705). The mechanisms of this effect are also unknown, but may be similar to the effect of T cell vaccination.

Binz and Wigzell immunized mice and rats with anti-MHC antibodies in complete Freunds adjuvant (CFA), or with purified MLR activated T lymphoblasts in CFA. They reported MHC-restricted reduction of MLR, cell mediated lymphotoxicity (CML), graft versus host disease (GVH), and prolongation of skin allograft survival, and shown induction of anti-idiotypic antibodies, as well as auto-anti-idiotypic killer and suppressor T cells (Anderson, L.C. et al., (1977) J.Exp.Med. **146**: 1124-1137; Binz H. et al., (1978) J.Exp.Med. **147**: 63-76). The present invention extended T cell vaccination to cardiac allo-transplantation as a model of a vascularized graft. The major difference between the results in Examples IX to XI here and those reported by Binz and Wigzell concern the degree of specificity: in contrast to the non-specific inhibition observed here, Binz and Wigzell reported no inhibitory effect on a third-party allograft following their procedure.

**Claims**

1. A composition useful for preventing or treating allergy or graft rejection in a subject comprising activated T lymphocytes, a membrane preparation thereof, a T cell receptor protein of said T lymphocytes, or a peptide of about 5-30 amino acids having a sequence corresponding to the sequence of part of said T cell receptor protein or a functional derivative of said peptide, wherein said T lymphocytes are either specific for the antigen provoking said allergy or responsive to any antigen provoking graft rejection.

2. The composition according to claim 1, wherein said T lymphocytes are obtained from the blood or a lymphatic organ of the subject or from a cultured T cell line or clone of the subject, and are activated **in vitro**.

3. The composition according to claim 2, wherein said T lymphocytes are activated by a polyclonal T cell activator.

4. The composition according to claim 1 or 2 for the prevention or treatment of allergy wherein said T lymphocytes are activated **in vitro** by the specific antigen provoking said allergy.

5. The composition according to claim 1 or 2 for the prevention or treatment of graft rejection wherein said T lymphocytes are activated **in vitro** by an alloantigen by culturing with allogeneic cells derived from the prospective donor or from any other third party.

6. The composition according to any one of claims 1 to 5 wherein said activated T lymphocytes have been subjected to a treatment which produces membrane aggregation selected from treatment with a chemical cross-linking agent, hydrostatic pressure, a cytoskeletal disrupting agent, or attenuation with X- or gamma-irradiation or with mitomycin C,or a combination thereof.

7. The composition according to claim 1 comprising a membrane preparation of said T lymphocytes, a T cell receptor protein of said T lymphocytes, or a peptide of about 5-30 amino acids having a sequence corresponding to the sequence of part of said T cell receptor protein, or a functional derivative thereof, and a pharmaceutically acceptable carrier.

8. A method for producing a composition according to claim 1 useful for the prevention or treatment of graft rejection in a subject, which composition comprises activated T lymphocytes optionally subjected to treatment which produces membrane aggregation, said T lymphocytes being responsive to any antigen provoking graft rejection, said method comprising:

(a) obtaining T lymphocytes or cells from said subject or from a cell culture;

(b) treating said T lymphocytes **in vitro** with an activating agent, thereby activating said T lymphocytes; and optionally

(c) treating said activated T lymphocytes with a chemical cross-linking agent, hydrostatic pressure, a cytoskeletal disrupting agent, gamma-irradiation, X-irradiation, or any combination thereof, thereby producing said composition.

9. A method for producing a composition according to claim 1 useful for the prevention or treatment of allergy in a subject, which composition comprises activated T lymphocytes optionally subjected to treatment which produces membrane aggregation, said T lymphocytes being specific for the antigen provoking said allergic reaction, said method comprising:

(a) obtaining said antigen-reactive T lymphocytes or cells from said subject or from a cell culture;

(b) treating said T lymphocytes **in vitro** with an activating agent, thereby activating said T lymphocytes; and optionally

(c) treating said activated T lymphocytes with a chemical cross-linking agent, hydrostatic pressure, a cytoskeletal disrupting agent, gamma-irradiation, X-irradiation, or any combination thereof, thereby producing said composition.

|       | Vaccine | |
| :---: | :---: | :---: |
| | I-LNC | Cell |
| Group | specificity | treatment |
| A | none | None |
| B | Naive | Glutaraldehyde |
| C | DNFB | Irradiated |
| D | DNFB | Glutaraldehyde |
| E | Irritation control | |

CS Response to DNFB

(50% suppression)

$\Delta$ Ear swelling x $10^{-2}$ mm

FIGURE 1

| | Vaccine | |
|---|---|---|
| Group | I-LNC specificity | Cell treatment |
| A | None | None |
| B | OX | Irradiated |
| C | OX | Glutaraldehyde |
| D | Irritation control | |

CS response to OX

(73% suppression)

$\Delta$ Ear swelling x 10$^{-4}$ inch

FIGURE 2

| Group | I -LNC(Ox) glutaraldehyde treated vaccine | Challenge antigen |
|---|---|---|
| A | No | OX |
| B | Yes | OX |
| C | No | DNFB |
| D | Yes | DNFB |
| E | Irritation control | OX |
| F | Irritation control | DNFB |

**CS Response**

(59% suppression)

$\Delta$ Ear swelling x 10$^{-4}$ inch

0    20    40    60    80

FIGURE 3

| | | Recipient mice | | CS response |
|---|---|---|---|---|
| Group | Donor l-LNC (OX+DNFB) | l-LNC(OX) vaccine | Challenge antigen | |
| A | Yes | None | OX DNFB | |
| B | Yes | Irradiated | OX DNFB | |
| C | Yes | Glutaraldehyde | OX DNFB | |
| D | Irritation control | | OX DNFB | |

Δ Ear swelling x 10 $^{-4}$ inch

(78% suppression)

FIGURE 4

| I-LNC(DNFB) vaccine | I-LNC Challenge specificity | |
|---|---|---|
| A. None | DNFB OX | |
| B. Irradiated | DNFB OX | |
| C. Glutaraldehyde | DNFB OX | |

Response to I-LNC

$\Delta$ Ear swelling x $10^{-4}$ inch

FIGURE 5

## MLR IN LEWIS RATS AFTER T-CELL VACCINATION
### (lymph nodes)

FIGURE 6a

## MLR IN LEWIS RATS AFTER T-CELL VACCINATION
### (Spleens)

FIGURE 6b

## MLR IN LEWIS RATS AFTER T-CELL VACCINATION
## WITH ANTI-WISTAR CELLS (Lymph nodes)

FIGURE 6c

## MLR IN BALB/C MICE AFTER T-CELL VACCINATION

FIGURE 7

## MLR IN C57BL/6 PRIMED BALB/C MICE

FIGURE 8

## INCREASED MLR AFTER ERGOTYPIC VACCINATION

FIGURE 9

## VACCINATION INDUCED SUPPRESSION

FIGURE 10

FIGURE 11

T Cell Vaccination Against Rejection of Heart Allo-graft

% Survival

Days

Control

T Cell Vaccination

## T Cell Vaccination Against Rejection of Heart Allo-graft

FIGURE 12

FIGURE 13

## DOCUMENTS CONSIDERED TO BE RELEVANT

EP 91116849.0

| Category | Citation of document with indication, where appropriate, of relevant passages | Relevant to claim | CLASSIFICATION OF THE APPLICATION (Int. Cl.5) |
|---|---|---|---|
| A | EP - A - 0 333 606 (YEDA RESEARCH & DEVELOPMENT COMPANY) * Claims 1-8 * -- | 1,6,8, 9 | A 61 K 35/12 A 61 K 39/00 A 61 K 37/02 |
| D,A | US - A - 4 634 590 (CHEN et al.) * Claims * -- | 1,6-9 | |
| D,A | SCIENCE, vol. 244, May 19, 1989 (Wahington DC) A.W. LOHSE et al "Control of Experimental Autoimmune Encephalomyelitis by T Cells Responding to Activated T Cells" pages 820-822 * Totality * ---- | 1 | |
| | | | TECHNICAL FIELDS SEARCHED (Int. Cl.5) A 61 K |

The present search report has been drawn up for all claims

| Place of search VIENNA | Date of completion of the search 23-12-1991 | Examiner WOLF |
|---|---|---|

EPO FORM 1503 03.82 (P0401)